# EUROPEAN PATENT SPECIFICATION

(11) **EP 1 019 524 B1**
(45) Date of publication and mention of the grant of the patent: **29.09.2004**
(21) Application number: 98903847.6
(22) Date of filing: 30.01.1998
(51) Int. Cl.: C12Q 1/00

(54) **METHOD FOR IDENTIFYING INDUCERS AND INHIBITORS OF PROGRAMMED CELL DEATH**
VERFAHREN ZUR IDENTIFIZIERUNG VON INDUKTOREN UND HEMMSTOFFEN DES PROGRAMMIERTEN ZELLTODS
PROCEDES D'IDENTIFICATION DES INDUCTEURS ET DES INHIBITEURS DE LA MORT CELLULAIRE PROGRAMMEE

(30) Priority: 31.01.1997 US 36997 P; 04.02.1997 US 795470
(43) Date of publication of application: 19.07.2000
(73) Proprietor: ARCH DEVELOPMENT CORPORATION, Chicago, IL 60637 (US)
(72) Inventor: ROIZMAN, Bernard, Chicago, IL 60637 (US); BIN, He, Chicago, IL 60637 (US)
(74) Representative: Forstmeyer, Dietmar, Dr. rer. nat., Dipl.-Chem.
(86) International application number: PCT/US1998/001808
(87) International publication number: WO 1998/033933

(56) References cited:
- WO-A-93/19591
- Neuroscience Letters, Volume 201, 1995, Alberto Alcázar et al, "Phosphorylation of initiation factor 2 alpha subunit and apoptosis in Ca2+ ionophore-treated cultured neuronal cells", page 215 - page 218, XP002900195

## Description

### BACKGROUND OF THE INVENTION

Programmed cell death, or apoptosis is an active cellular mechanism and has several important implications. First, it is clear that such an active process can provide additional means for regulating cell numbers as well as the biological activities of cells. Secondly, mutations or cellular events which potentiate apoptosis may result in premature cell death. Third, a form of cell death that is dependent on a specific active cellular mechanism can at least potentially be suppressed. Finally, inhibition of programmed cell death may be expected to lead to aberrant cell survival and could be expected to contribute to oncogenesis, while conversely it is thought that tumor cell suicide may be induced through apoptosis or programmed cell death.

In general, programmed cell death involves distinctive morphological changes including nuclear condensation and degradation of DNA to oligonucleosomal fragments. In certain circumstances it is evident that apoptosis is triggered by or is preceded by changes in protein synthesis. For example, cellular protein synthesis may be significantly down-regulated. The DNA degradation described above may be a slow process, occurring days after the cessation of the cell's biosynthetic activities. Apoptosis appears to provide a very clean process for cellular destruction, in that the cells are disposed of by specific recognition and phagocytosis prior to bursting. In this manner cells can be removed from a tissue without causing damage to the surrounding cells. Thus, it can be seen that programmed cell death is important to a number of physiological processes, including morphological development, clonal selection in the immune system, and normal cell maturation and death in other tissue and organ systems.

It has also been demonstrated that cells can undergo apoptosis or programmed cell death in response to environmental stimuli. Examples include the appearance of a stimulus, such as glucocorticoid hormones for immature thymocytes, or the disappearance of a stimulus, such as interleukin-2 withdrawal from mature lymphocytes, or the removal of colony stimulating factors from hemopoietic precursors (for a review of literature see Williams, *Cell, 65*:1097-1098 [1991]). Furthermore, it has recently been demonstrated that the response to removal of nerve growth factor from established neuronal cell cultures that mimics target removal, or axiotomy, or other methods of trophic factor removal, is a triggering of a suicide program or programmed cell death. [See Johnson *et al*., *Neurobiol. of Aging, 10*:549-552 (1989)]. The authors proposed a "death cascade" or "death program," which envisions that trophic factor deprivation initiates the transcription of new mRNA and the subsequent translation of that mRNA into death associated proteins which act in sequence to ultimately produce "killer proteins." Such an intracellular mechanism seems to fit well with the characteristics of apoptosis discussed above, *e*.*g*., death of specific cells without the release of harmful materials and without the disruption of tissue integrity. Furthermore, the authors indicate that inhibitors of macromolecular synthesis prevented the death of neurons in the absence of nerve growth factor.

Studies have been conducted to explore the possibility that tumor cells could be eliminated by artificially triggering apoptosis. The anti-APO-1 monoclonal antibody induces apoptosis in several transformed human B and T cell lines. The antibody binds to a 52kd surface protein and could act either by mimicking a positive death-inducing signal or by blocking the activity of a factor required for survival. Anti-FAS antibodies have similar effects. The recent cloning and sequencing of the gene for the FAS antigen has shown that it is a 63 kilodalton transmembrane receptor. Itoh *et al*., *Cell, 66*:233-243 (1991).

However, neither APO-1 nor FAS is likely to function exclusively as a trigger for cell death. Both are cell surface receptors that may activate quite different cellular responses under other circumstances. Moreover, these antigens are not confined to tumor cells and their effect on normal cells is certainly an important consideration, as is the possible appearance of variants that no longer display the antigens.

It has also been demonstrated that the cell death induced by a range of cytotoxic drugs, including several used in cancer therapy, has also been found to be a form of apoptosis [Barry et al, *Biochem. Biopharmacol*., ***40***:2353-2362 (1990)]. This is also true, in many cases, for cell death after γ- or x-irradiation [Williams, *Cell, 65*:1097-1098 (1991)]. In fact, the failure of apoptosis or programmed cell death in tumor cells could be of fundamental importance in contributing not only to the evasion of physiological controls on cell numbers, but also to resistance both to natural defenses and to clinical therapy.

The expression of the bcl-2 gene has been shown to inhibit death by apoptosis. The bcl-2 gene was isolated from the breakpoint of the translocation between chromosomes 14 and 18 found in a high proportion of the most common human lymphomas, that being follicular B cell lymphomas. The translocation brings together the bcl-2 gene and immunoglobulin heavy chain locus, resulting in an aberrantly increased bcl-2 expression in B cells. Subsequently, Henderson *et al*., [*Cell*, *65*:1107-1115 (1991)] demonstrated that expression of latent membrane protein 1 in cells infected by Epstein-Barr virus protected the infected B cells form programmed cell death by inducing expression of the bcl-2 gene. Sentman *et al*. [*Cell*, *67:* 879-888 (1991)] demonstrated that expression of the bcl-2 gene can inhibit multiple forms of apoptosis but not negative selection in thymocytes. Strasser *et al*. [*Cell, 67*:889-899 (1991)] demonstrated that expression of a bcl-2 transgene inhibits T cell death and can perturb thymic self-censorship. Clem *et al.* [*Science, 245*:1388-1390 (1991)] identified a specific baculovirus gene product as being responsible for blocking apoptosis in insect cells. Gagliardini et al [*Science, 263:*826-828 (1994)] demonstrated the ability of the *crm*A gene product to prevent apoptosis in the dorsal root ganglion cells of chicken which had been deprived of nerve growth factor. More generally, Barinaga, [*Science, 263*:754-756 (1994)] also discusses the role of bc1-2, Bax(long), bclX(short) and ICE in apoptosis.

A number of diseases have been associated with apoptosis of neuronal cells. For example, amyotrophic lateral sclerosis (Lou Gehrig's disease) has been associated with apoptotic cell death. Alexianu *et al*., *J*. *Neurochem.* **63**:2365-2368 (1994). Spinal muscular atrophy is associated with the partial deletion of an apoptosis inhibitory protein which results in apoptotic cell death. Roy *et al*., *Cell* **80**:167-178 (1995). Huntington's disease has also been associated with apoptotic cell death. Portera-Cailliau *et al*., *J. Neurosci* **15**:3775-3787 (1995). Apoptotic cell death has also been strongly implicated in Alzheimer's disease, Gschwind *et al*., *J*. *Neurochem* **65**:292-300 (1995); and LaFerla *et al., Nat*. *Genet.* **9**:21-30 (1995).

Thus it is clear that the ability to control apoptosis or programmed cell death, such as by inducing it to occur in cells (*e.g.* in tumor cells), or to prevent apoptosis or programmed cell death (*e*.*g*. in neurodegenerative conditions such as Alzheimer's disease, amyotrophic lateral sclerosis, Huntington's disease, spinal muscle atrophy, and other neurodegenerative disorders) will allow therapeutic intervention in diseases for which there are currently few if any therapeutic modalities. In order to achieve such therapeutic control, screening methods must be made available for identifying substances which induce or inhibit programmed cell death.

### SUMMARY OF THE INVENTION

It is an object of the invention to provide methods for identifying inducers and inhibitors of programmed cell death in cell-free systems. More particularly, the invention is directed to methods for identifying inhibitors or inducers of programmed cell death or apoptosis which exert their effects by interacting with elements of the protein synthetic machinery of a cell when tested in a cell-free system.

The invention exploits the observation that cells whose protein synthesis is shut down as the result of certain stresses that provoke programmed cell death or apoptosis, exhibit increased ability to phosphorylate or to prevent dephosphorylation of eIF-2α. The invention also expolits the observation that proteins which have been shown to prevent programmed cell death such as γ₁34.5 and GADD34 reduce the level of phosphorylation of eIF-2α

According to the invention, an inhibitor of programmed cell death is identified by its ability to decrease the level of phosphorylation of phosphorylated eIF-2α or to prevent phosphorylation of eIF-2α in a cell-free system comprising PP1α and eIF-2α.

Inducers of programmed cell death are identified in a cell-free system of the invention by their ability to prevent the dephosphorylation of eIF-2α, to induce the phosphorylation of eIF-2α and/or to interact with (e.g. activate) PKR.

Other objects and advantages of the invention may be apparent to those skilled in the art from review of the following detailed description, taken in conjunction with the figures and the appended claims.

### BRIEF DESCRIPTION OF THE FIGURES

Figures 1A and 1B illustrate eIF-2α kinase activity in infected cells and mock infected cell extracts.
Figures 2A and 2B illustrate the association of phosphatase 1α with γ₁34.5 and MyD116.
Figures 3A and 3B show autoradiographic images of electrophoretically separated ³⁵S-methionine labelled proteins from cell lysates infected with the indicated viruses in the presence or absence of okadaic acid.
Figure 4 depicts autoradiographic images of electrophoretically separated eIF-2 after reaction with variants of cells mock infected, and of cells infected with HSV-1(F), R3616, and R8300.

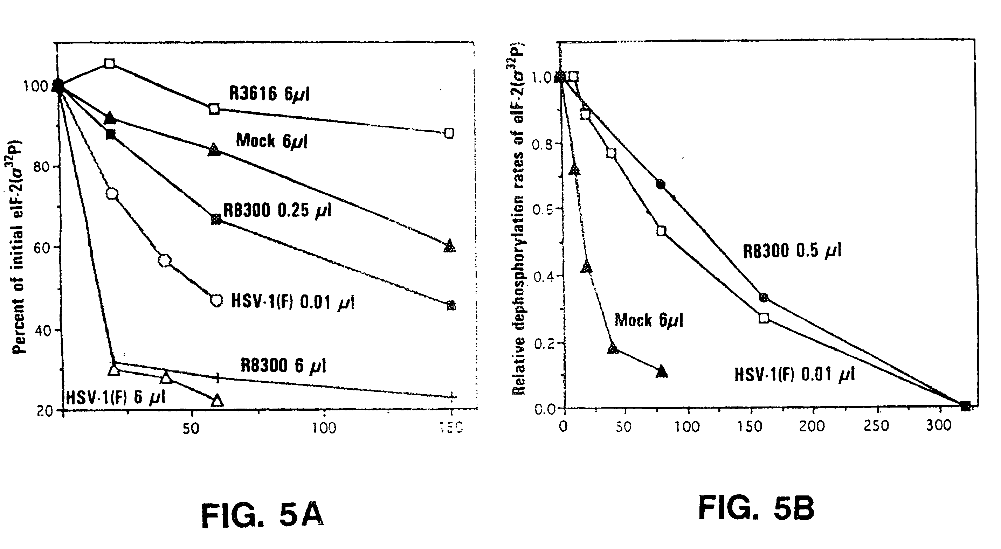

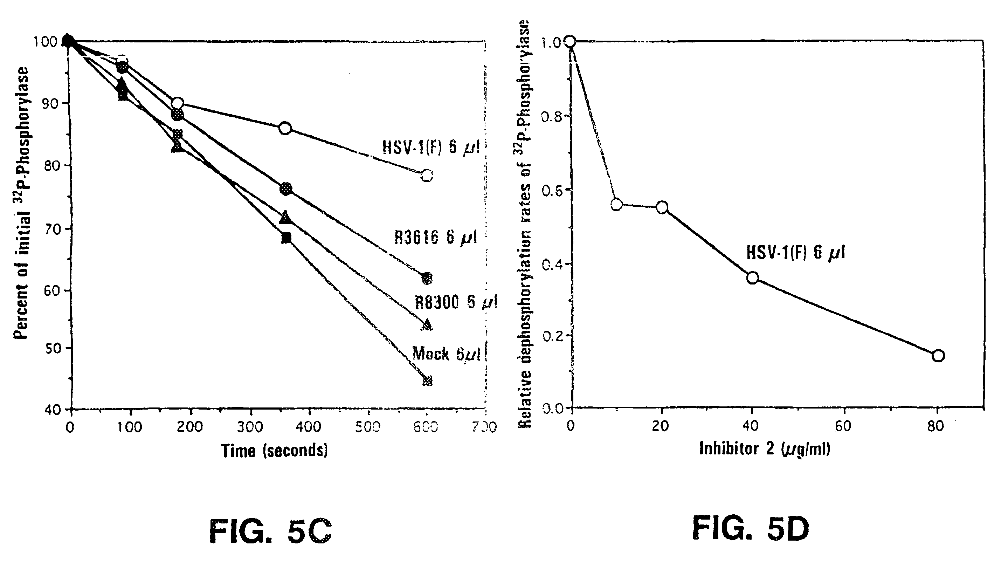

Figures 5A through 5D illustrate phosphatase activity in S10 fractions of HeLa cells mock-infected, and of such cells infected with 20 pFU of HSV-1(F), R3616, or R8300 per cell. Figure 5A shows residual radio-activity in eIF-2α after reaction with variable amounts of S10 fractions from mock-infected or infected cells. Figure 5B shows the effect of inhibitor 2 on the rate of dephosphorylation of eIF-2α 1α³²P by the phosphatase activity in the S10 fractions. Figure 5C shows ³²P-phosphorylase phosphatase activity. Figure 5D shows the effect of inhibitor 2 on the relative rate of ³²P-phosphorylase phosphatase activity.

### DETAILED DESCRIPTION

Herpes simplex viruses have particular characteristics that make them useful for the study of programmed cell death (see, WO 93/19591 published 14 October 1993 and references cited below). Herpes simplex virus 1 (HSV-1) encodes a gene, γ₁34.5, whose function is to preclude a host response which terminates all protein synthesis subsequent to the onset of viral DNA synthesis [Chou *et al*., *Proc*. *Natl*. *Acad. Sci*. *USA* **89**:3266-3270 (1992)]. The γ₁34.5 gene maps in the sequences flanking the long unique sequence of HSV-1 DNA and therefore is present in two copies per genome [Chou *et al*., *J. Virol*. **57**:629-637 (1986)]. The 263 amino acid protein encoded by the HSV-1(F) γ₁34.5 consists of three domains, an amino terminal domain of 160 amino acid-domain, ten repeats of three amino acids (AlaThePro), and a 73 amino acid-carboxyl terminal domain [Chou *et al*., *J. Virol*. **64**:1014-1020 (1990)]. A stretch of 64 amino acids at the carboxyl terminus of the γ₁34.5 is homologous to a corresponding stretch of amino acids of the carboxyl terminus of a murine protein known as MyD116 and a Chinese hamster protein known as GADD34 [Chou *et al*., *Proc*. *Natl*. *Acad. Sci. USA* **89**:3266-3270 (1992); McGeoch *et al*., *Nature (London)* **353**:609 (1991)]. MyD116 is a member of a set of proteins induced in myelogenous leukemia cells induced for terminal differentiation by interleukin 6 [Lord *et al*., *Nucleic Acids Res.* **18**:2823 (1990)]. GADD34, structurally closely related to MyD116, is also one of a subset of proteins induced following DNA damage or cell growth arrest [Fornace *et al*., *Mol. Cell. Biol*. **9**:4196-4203 (1989); Fornace *et al*., *Ann*. *N. Y*. *Acad. Sci.* **663**:139-153 (1992); Zhan *et al*., *Mol. Cell. Biol.* **14**:2361-2371 (1994)].

Infection of human cells with herpes simplex virus in which both copies of γ₁34.5 are inactivated or deleted, but particularly of human neuroblastoma cell line SK-N-SH or primary human foreskin fibroblasts results in nearly complete cessation of the host cell protein synthesis and the replicative cycle of the virus [Chou *et al*., *J. Virol*. **66**:8304-8311 (1994)]. This total premature shutoff of protein synthesis is not seen in these cells when they are treated with inhibitors of viral DNA synthesis or in Vero cells [Chou *et al*., *Proc. Natl*. *Acad. Sci. USA* **89**:3266-3270 (1992)].

The capacity to preclude total premature shutoff of protein synthesis maps in the carboxyl terminus domain of γ₁34.5 protein that is homologous to the MyD116 protein [Chou *et al*., *Proc. Natl. Acad. Sci*. *USA* **91**:5247-5251 (1994)]. Indeed, the carboxyl terminus of MyD116 successfully substitutes for the corresponding domain of γ₁34.5. The viruses lacking the γ₁34.5 protein or unable to express the carboxyl terminus of protein are totally avirulent in a murine encephalitis model of HSV-1 infections [Chou *et al*., *Science* **250**:1262-1266 (1990); Whitley *et al*., *J*. *Clin*. *Invest.* **91**:2837-2843 (1993); McKie *et al*., *J*. *Gen. Virol*. **75**:733-741 (1994)].

Chou *et al*., *Proc*. *Natl*. *Acad. Sci. USA* **92**:10516-10520 (1995) extended these observations and demonstrated that in wild-type or mutant infected cells, the double strand RNA-dependent protein kinase (PKR) was activated. However, only in cells infected with the γ₁34.5-mutants lacking the γ₁34.5 domain encoding the carboxyl terminus of the protein was the a subunit of eIF-2α phosphorylated.

The premature shut off of protein synthesis associated with programmed cell death raises several interesting questions, i.e. what triggers the host response, what is the mechanism by which protein synthesis is turned off, and what is the mechanism by which γ₁34.5 precludes the host response.

The data set out below shows that (i) the carboxy terminus of MyD116 interacts with protein phosphatase 1α in yeast, and both MyD116 and γ₁34.5 interact with protein phosphatase 1α *in vitro;* (ii) protein synthesis in infected cells is strongly inhibited by okadaic acid, a phosphatase 1 inhibitor, and (iii) the α subunit in purified eIF-2 phosphorylated *in vitro* is specifically dephosphorylated by S10 fractions of wild-type infected cells at a rate 3000 times that of mock-infected cells, whereas the eIF-2α-P phosphatase activity of γ₁34.5⁻ virus infected cells is lower than that of mock-infected cells. The eIF-2α-P phosphatase activities are sensitive to protein phosphatase Inhibitor 2. In contrast to eIF-2α-P phosphatase activity, extracts of mock-infected cells exhibit a 2-fold higher phosphatase activity on [³²P] phosphorylase than extracts of infected cells. These results indicate that in infected cells, γ₁34.5 interacts with and redirects phosphatase to dephosphorylate eIF-2α to enable continued protein synthesis despite the presence of activated PKR. The GADD34 protein may have a similar function in eukaryotic cells. The mechanism for maintenance of protein synthesis in the face of double-stranded RNA accumulation is different from that described for viruses examined to date.

Placing the foregoing observations into a broader context, in cells infected with HSV-1 accumulation of symmetric transcripts after the onset of DNA synthesis results in activation of PKR. A viral protein, γ₁34.5 binds PP1α, presumably through its carboxy terminus, and redirects the activity of the enzyme to dephosphorylate eIF-2α either by itself or in conjunction with other proteins. As a consequence, although PKR is activated, protein synthesis continues unabated. The following data support this scenario.

In cells infected with both wild-type and γ₁34.5⁻ mutants, PKR is phosphorylated but only in cells infected with mutants lacking all or the 3' domain of the γ₁34.5 gene is protein synthesis shut off and eIF-2α phosphorylated. The γ₁34.5 sequence required to block protein synthesis shut off maps in the 3' domain of the gene and can be replaced without loss of function by the corresponding domain of MyD116, the murine GADD34 gene.

The eIF-2α-P phosphatase activities reported here may account for the differences in steady-state eIF-2α-P and protein synthesis previously noted in γ₁34.5⁻ virus-infected cells [Chou *et al*., *Proc. Natl*. *Acad. Sci*. *USA* **92**:10516-10520 (1995)]. Because PKR is not activated in mock-infected cells, the observed moderate eIF-2α-P phosphatase activity should be sufficient to prevent the degree of phosphorylation of eIF-2α required to inhibit protein synthesis. Wild-type HSV-1 infection does result in activation of PKR [Chou *et al*., *Proc*. *Natl*. *Acad. Sci. USA* **92**:10516-10520 (1995)], but its effect is probably more than countered by the ∼ 3000-fold increase in eIF-2α-P phosphatase. This marked increase in eIF-2α-P phosphatase activity is due to the expression of the γ₁34.5 gene product, because infection with the γ₁34.5⁻ mutant is associated with a decrease in eIF-2α-P phosphatase activity, that, upon activation of PKR, results in phsophorylation of eIF-2α and inhibition of protein synthesis [Chou *et al*., *Proc. Natl. Acad. Sci. USA* **92**:10516-10520 (1995)].

In the yeast two-hybrid system the carboxyl terminus of MyD116 interacted with the human PP1α. The interaction of PP1α and MyD116 or with γ₁34.5 protein was also demonstrable in pulldown experiments *in vitro*. The hypothesis that the eIF-2α phosphatase activity measured in cytoplasmic fractions of mock-infected or infected cells is derived from PP1 is strengthened by the observation that it was sensitive to inhibitor 2. Nevertheless, two lines of evidence suggest that this activity may represent a modified form of PP1α redirected to eIF-2α. Specifically, the eIF-2α-P phosphatase activity was less sensitive to inhibitor 2 than that of mock-infected cells, and furthermore, the phosphorylase phosphatase activity, a known function of PP1, was more potent in uninfected cells than in infected cells.

This work also sheds light on the putative function of GADD34. As noted above, the carboxyl terminus of the murine GADD34 gene (MyD116) is homologous to and can substitute for the corresponding domain of γ₁34.5 [He *et al*., *J*. *Virol*., **70**:84.-90 (1996)]. It has also been shown that PP1α interacted with both γ₁34.5 and MyD116 chimeric proteins *in vitro* (see below). These results argue that at least one of the functions of the GADD34 gene is similar to that of the γ₁34.5 gene, and that the γ₁34.5 gene may be useful in replacing its mammalian cell homolog to sustain protein synthesis in the face of stress that would naturally abolish protein synthesis by phosphorylation of eIF-2α.

Lastly, most viruses studied to date block phosphorylation of eIF-2α by encoding proteins which bind double stranded RNA, *e.g.,* E2L of vaccinia [Davies *et al*., *J*. *Virol.* **67**:1688-1692 (1993); Carroll *et al*., *J*. *Biol. Chem*. **268**:12837-12842 (1993); and Yuwen *et al*., *Virology* **195**:732-744 (1993)], α3 protein of Reo and rotaviruses [Langland *et al*., *J*. *Virol*. **68**:3821-3829 (1994); Beattie *et al., J. Virol*. **69**:499-505 (1995); Imani *et al*., *Proc*. *Natl. Acad. Sci. USA* **85**:7887-7891 (1988); and Lloyd *et al*., *J*. *Virol*. **66**:6878-6884 (1992)], and NS1 protein of influenza [Lu *et al*., *Virology* **214**:222-228 (1995)], or proteins or RNA that inactivate PKR by binding to it, *e.g.*, vaccinia K3L, Epstein-Barr virus EBERS1 and 2 [Sharp *et al*., *Nucleic Acids Res.* **21**:4483-4490 (1993)], adenovirus VA1 RNA [Sharp *et al*., *Nucleic Acids Res.* **21**:4483-4490 (1993); and Ghade *et al*., *J. Virol*. **68**:4137-4151 (1994)], influenza virus-induced cellular p58 protein [Lee *et al*., *Mol*. *Cell. Biol.* **14**:2331-2342 (1994)] or degrade PKR (pollovirus) [Black *et al*., *J. Virol*. **67**:71-800 (1993)]. HIV-1 TAR sequence appears to bind to a cellular TAR binding protein and PKR [McMillan *et al*., *Virology* **213**:413-424 (1995); Park *et al*., *Proc*. *Natl. Acad*. *Sci*. *USA* **91**:4713-4717 (1994); and Consentino *et al*., *Proc. Natl. Acad. Sci*. *USA* **92**:9445-9449 (1995)]. The exceptions may be hepatitis delta virus that does not activate PKR even though *in vitro* it consists largely of double-stranded RNA [McNair *et al*., *J. Gen. Virol*. **75**:1371-1378 (1994)], and simian virus 40 whose large T antigen acts at a step postactivation of PKR [Swaminahan *et al*., *J*. *Virol*. **219**:321-323 (1996)]. These data suggest that HSV-1 and HSV-2 use a very different mechanism to preclude the shutoff of protein synthesis by targeting phosphorylated eIF-2α rather than either PKR or double-stranded RNA.

The elucidation of these mechanisms provides the ability to develop new cell-free methods which are useful for the screening of both inducers and inhibitors of programmed cell death, such inhibitors or inducers of programmed cell death may provide useful therapeutic agents for the prevention of diseases associated with the induction of programmed cell death as well as therapeutics for diseases such as any of a wide variety of tumorigenic diseases in which it would be desirable to induce programmed cell death. Such systems are also useful for identifying antiviral substances which influence programmed cell death in infected cells by mechanisms discussed herein and as exemplified below. The following examples exemplify the practice of the invention and are not intended to limit the scope of the invention as recited in the claims.

Example 1 describes the cells and viruses used in the studies.

Example 2 demonstrates that the phosphorylation of eIF-2α is associated with a kinase present in a fraction enriched for ribosomes of cells infected with R3616 (γ₁34.5⁻) and in which protein synthesis was shut off. These data also show that immunoprecipitation of PKR from the same fraction after reaction with [γ³²P]-ATP yielded several phosphorylated polypeptides.

Example 3 describes the cloning and expression of human protein phosphatase 1α.

Example 4 describes the measurement of eIF-2α phosphatase activity.

Example 5 describes the determination of phosphorylase and phosphatase activity.

Example 6 describes the interaction of GADD3y (MyD116) with PP1α in a yeast two-hybrid system, and the identification of PP1α.

Example 7 describes the binding of MyD116 and γ₁ 34.5 to PP1α *in vitro*.

Example 8 describes the role of phosphatase activity in protein synthesis in infected cells.

Example 9 describes the activity of eIF-2 α phosphatase in wild type or R8300 recombinant virus infected cells.

Example 10 describes the sensitivity of eIF-2α phosphatase activity to PPα Inhibitor 2.

Example 11 demonstrates that activated phosphatase activity is specific for eIF-2α.

Example 12 describes methods for identifying inducers and inhibitors of programmed cell death in cell-free systems.

### Example 1

### CELLS AND VIRUSES

Vero, HeLa cells, and the human neuroblastoma cells (SK-N-SH) from American Type Culture Collection, 12301 Parklawn Drive, Rockville, Maryland 20852 were propagated in Dulbecco's modified Eagle's medium supplemented with 5% (Vero and HeLa cells) and 10% (SK-N-SH cells) fetal bovine serum, respectively. HSV-1(F) is the prototype HSV-1 strain used in these studies (Ejercito *et al*., *J*. *Gen. Virol*. **2**:357-364 (1968)). The HSV-1 recombinant R3616 has been previously described [Chou *et al*., *Proc. Natl. Acad. Sci. USA* **89**:3266-3270 (1992); Chou *et al*., *Proc. Natl*. *Acad. Sci. USA* **91**:5247-5251 (1994); and Chou *et al*., *Science* **250**:1262-1266 (1990)]. R3616 virus lacks 1 Kb from the coding domains of both copies of γ₁34.5 genes. In recombinant virus R8300, the sequence encoding the carboxyl terminal domain of the γ₁ 34.5 gene was replaced with the corresponding domain of the MyD116 gene as described in He *et al*., *J. Virol*. **70**:84-90 (1996).

### Example 2

### AN eIF-2α KINASE ACTIVITY IS ASSOCIATED WITH THE RIBOSOMAL FRACTION IN CELLS INFECTED WITH γ₁34.5⁻ VIRUS

In view of the results described in Chou *et al*., *Proc*. *Natl*. *Acad. Sci. USA* **92**:10516-10520 (1995), a series of studies was conducted to determine whether the premature shutoff of protein synthesis in human neuroblastoma cells infected with R3616 is due to modification (phosphorylation) of a subunit of the translation initiation factor eIF-2 via eIF-2α kinase activity. The experiments utilized HeLa cells inasmuch as eIF-2 appears to be more stable in lysates of this cell line. HeLa cells, like most human cell lines studied to date, are also affected by premature shutoff of protein synthesis in cells infected with γ₁34.5⁻ viruses.

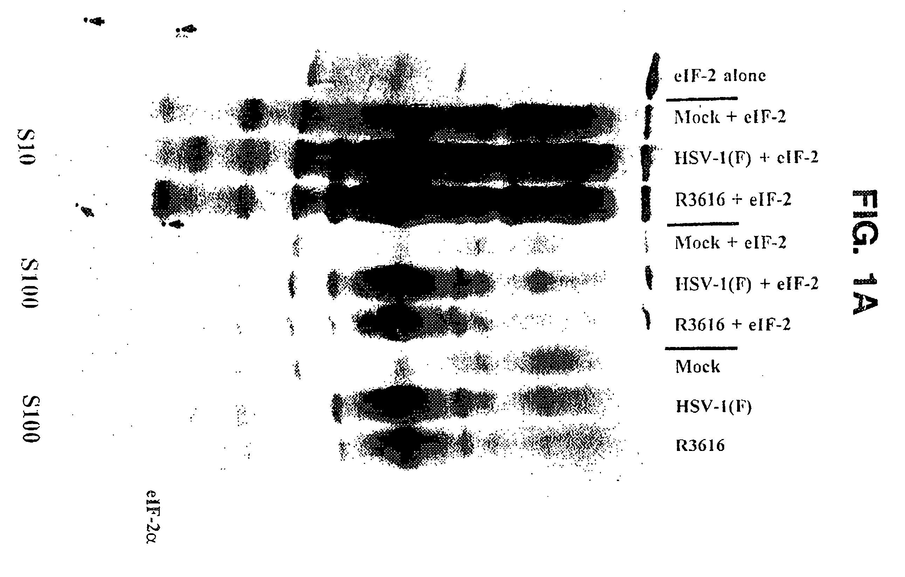

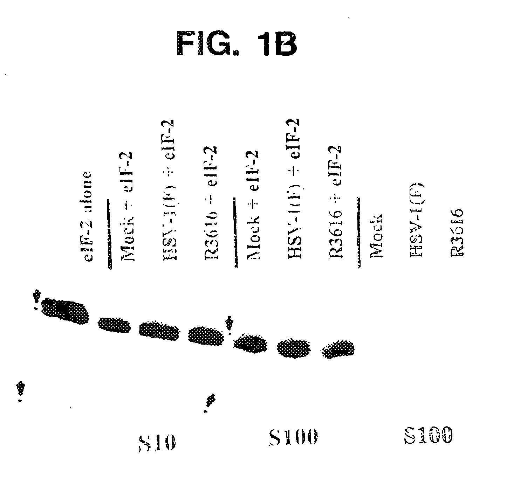

Replicate cultures of HeLa cells were either mock infected or infected with HSV-1(F) or R3616 viruses as described in Chou *et al*., *Proc. Natl. Acad. Sci. USA* **92**:10516-10520 (1995). At seven hrs after infection the cells were harvested and S10 fractions, which includes all cytoplasmic materials (excluding mitochondria) were prepared according to procedures described by Pollard and Clemens (Pollard *et al*., *In Methods in Molecular Biology: New Nucleic Acid Techniques* (Walker, J.M., ed.), Chapter 5, pp.47-60, Humana Press, Clifton, N.J. (1988)). S100 fractions (post ribosomal) were supernatant fluids prepared from S10 fractions after centrifugation at 29,000 rpm for three hrs at 4°C in a Beckman SW41 rotor. This fraction contained most of the soluble proteins but was free of ribosomes. Rabbit eIF-2 was purified from rabbit reticulocyte lysate according to the method describe by Grass *et al*., *J*. *Biol. Chem*., ***255***:6270-6275 (1980) was used to detect the eIF-2α kinase activity present in those extracts. 0.2µg of eIF-2 was reacted with the S10 or S100 fractions in the presence of [γ³²P]-ATP (100 µCi per sample, Sp. act. 6000 Ci/mmol., NEN, Boston, MA) at 30°C for 20 minutes. The reaction mixtures were then solubilized, electrophoretically separated on a denaturing 12 % polyacrylamide gel crosslinked with N'N'-diallyltartardiamide, transferred unto nitrocellulose sheets and set for autoradiography as previously described (Chou *et al*., *Proc*. *Natl. Acad. Sci*. *USA* **89**:3266-3270 (1992)). Figure 1 shows the results of the analysis. Lanes with exogenous eIF-2 added were indicated on the extreme left. The position of eIF-2α is shown on the right.

As shown in Figure 1, Panel A, eIF-2α subunit was extensively phosphorylated in the S10 extracts of R3616 infected cells, minimally phosphorylated in wild type- or mock-infected cells, and not phosphorylated at all in the absence of cell extracts. These results indicate that the S10 fraction of the γ₁34.5⁻ virus-infected cells contained an eIF-2a kinase activity that was much reduced or absent in similar extracts of mock-infected or wild type-infected cells. This activity was absent from all S 100 fractions tested suggesting that the kinase activity is associated with ribosomes. In the absence of exogenous eIF-2, minimal phosphorylation of endogenous eIF-2 was observed in all extracts due to steady state phosphorylation and de-phosphorylation processes in the cell.

Figure 1, Panel B shows a photograph of a nitrocellulose sheet containing separated proteins stained with monoclonal antibody specific to eIF-2α (Scorsone *et al*., *J. Biol*. *Chem.* **262**:14538-14543 (1987)). After reacting with appropriate secondary antibody, the blot was developed with color reagents provided by Promega (Madison, Wisconsin) to identify the eIF-2α polypeptide. Arrows indicate the dots used to orient and align the phosphorylated eIF-2α polypeptide in panel A with the antibody stained eIF-2α polypeptide in Panel B. The data in panel B of Figure 1 show that the level of eIF-2α was similar in all samples to which it had been added.

This activity was not present in mock-infected or wild type-infected cells. eIF-2 binds Met-tRNA^{f} and GTP in a ternary complex which then associates with ribosomal subunits in a pre-initiation complex (Merrick, W.C., *Microbiological Reviews* **56**:291-315 (1992); Hershey, J.W.B., *Annu. Rev. Biochem.* **60**:717-755 (1991)). After initiation, eIF-2 is recycled off the 80S complex with the help of eIF-2B, another translational factor. One of the events that regulates translation is the phosphorylation of the α subunit of the eIF-2 complex. Phosphorylation of eIF-2α correlates with shut off of protein synthesis in heme-regulated hemopoietic cells, in response to growth inhibition, cellular stresses caused by virus infections, heat shock, heavy metals, deprivation of serum, amino acids, or glucose (Hershey, J.W.B., *Annu. Rev. Biochem.* **60**:717-755 (1991); Sarre, T., *BioSystems* **22**:311-325 (1989)). These effects have been linked to the phosphorylation of the ser₅₁ of eIF-2α either by PKR, a Mᵣ 68,000 a kinase also know as dsl, a double stranded RNA activated eIF-2α kinase which autophosphorylates itself, or by HRI, a heme regulated eIF-2α kinase (Merrick, W.C., *Microbiological Reviews* **56**:291-315 (1992); Hershey, J.W.B., *Annu. Rev. Biochem.* **60**:717-755 (1991)). A primary role of the phosphorylation of ser₅₁ in the regulation of protein synthesis is supported by the observation that substitution of the ser₅₁ with Ala precludes phosphorylation and maintains protein synthesis (Pathak *et al*., *Mol Cell Biol*. **8**:993-995 (1988)).

### Example 3

### CLONING AND EXPRESSION OF HUMAN PROTEIN PHOSPHATASE α (PP1α)

The plasmid pRB4891, containing a 1.37 Kb cDNA encoding human protein phosphatase 1α (PP1α) was isolated from a commercially available human brain cDNA library (Stratagene). To construct pRB4890, a BamHI-EcoRI DNA fragment encoding codons 524 to 657 of MyD116 was amplified by PCR from a cDNA copy of the gene described by He *et al*., *J. Virol*. **70**:84-90 (1996) with the primers TGACTGGATGCAGAGGCGGCTCAGATTGTTC (SEQ ID NO. 1) and AGCGCGCAATTAACCCTCACTAAAG (SEQ ID NO. 2) and inserted into the BamHI-EcoRI sites of pGBT9 (Clontech) to serve as "bait" in the two-hybrid system. The PCR conditions were 94°C 2 min, 60°C 3 min and 72°C 3 min for 25 cycles. pRB4892 containing the chimeric GST-PP1α gene was constructed by ligating a 0.9 Kb PCR fragment containing the entire coding sequence of PPla except the first methionine codon into the EcoRI-SalI sites of pGEX4T-1 (Pharmacia). The oligonucleotide primers were GCACTGAATTCTCCGACAGCGAGAAGCTCAAC (SEQ ID NO. 3) and GCACTGTCGACATCTGGGGCACAGGGTGGTGT (SEQ ID NO. 4). To construct pRB4893 carrying a chimeric glutothione S transferase (GST)-carboxyl terminal domain of γ₁34.5 [GST-γ₁34.5(C)], the EcoRI-SalI fragment encoding codons 146 to 263 of γ₁34.5 from pRB71 and cloned into the EcoRI-SalI sites of pGEX4T-1. The construction of pRB4873 containing an AccI-EcoRI fragment encoding the 3' terminal 174 codons of MyD116 fused in frame to GST [GST-MyD116(C)] was as reported by He *et al*., *J*. *Virol*. **70**:84-90 (1996). Expression of GST-MyD116(c), GST-γ₁34.5(C) and GST-PP1 fusion proteins was induced by the addition of isopropyl-β-D-thiogalactoside to the medium with *Escherichia coli* BL21 cells transformed with either plasmid pRB4873, pRB4892 or pRB4892, followed by affinity purification of the fusion proteins from bacterial lysates on agarose beads conjugated with glutathione.

### Example 4

### DETERMINATION OF eIF-2α PHOSPHATASE ACTIVITY

The translation initiation factor eIF-2 was purified from rabbit reticulocyte ribosomes according to a method of Gross *et al*., *J. Biol*. *Chem*. **255**:6270-6275 (1980). A hemin-controlled translational repressor (HCR) was partially purified to step 4 from rabbit reticulocyte lysates according to the method described by Gross *et al*., *Biochem*. *Biophys. Res. Comm*.**50**:832-838 (1983). eIF-2 (20 pmol or 2.7 µg) was reacted with step 4 HCR in 0.02 M Tris-HCl, pH 7.5, 40 mM KCl, 2.0 mM MgCl₂, and 0.17 mM [γ³²P] ATP (2-10 Ci or 74-370 GBq/mmol) in a final volume of 12 µl for 25 min at 34°C to yield phosphorylated eIF-2α and eIF-2β (1.0 and 0.7 mol/mol of eIF-2, respectively). HeLa cells were harvested 15 hrs after mock infection or infection with 20 PFU of HSV-1(f), R3616 or R8300 per cell. S 10 fractions were prepared from lysates of mock-infected or infected cells as described by Chou *et al*., *Proc*. *Natl*. *Acad. Sci. USA* **92**:10516-10520 (1995) and diluted to a final volume of 18 µl with 0.02 M Tris-HCl, pH 7.5, 50 mM KCl, 2 mM MgCl₂, and 0.1 mM EDTA. ATP was then added to a final concentration of 0.8 mM. After 30 seconds at 34°C, each sample received 1.2 µl (2 pmol) of eIF-2(α³²P) and was reincubated at 34°C. The rate of dephosphorylation of eIF-2(α³²P) was determined by a method according to Gross *et al*., *Biochim. Biophys. ACTA* **740**:255-263 (1983) by placing 6.0 µl aliquots into a solution containing SDS at times indicated in the figures, followed by electrophoresis on 7% denaturing polyacrylamide gels as described for 21 hrs at 44 V (3 V/cm), staining with Coomassie Blue, or Silver, drying, and autoradiography. ³²P remaining in eIF-2(α³²P) was quantified by excising this band and comparing its Cerenkov radiation to that of equivalent aliquotes of eIF-2(α³²P) that were not further incubated and were subjected to electrophoresis in parallel.

### Example 5

### DETERMINATION OF PHOSPHORYLASE AND PHOSPHATASE ACTIVITY

Phosphorylase b (32 µg, Sigma) was phosphorylated by incubation with 3.3 µg of phosphorylase kinase (Sigma, St. Louis, MO) in 0.01 M Tris-HCl, pH7.5, 2.0 mM MgCl₂, and 0.13 mM [γ³²P] ATP (3 Ci or 111 GBq/mmol) in a final volume of 15 µl at 34°C for 10 min. Dephosphorylation was determined by adding 4.4 µg of [³²P] phosphorylase to samples constituted and incubated as described in Example 4 in a final volume of 27 µl. At 1.5, three, six and ten min., 6.0 µl aliquotes were removed and processed as described above for determining phosphatase activity.

### Example 6

### THE CARBOXYL TERMINUS OF THE MURINE GADD34 PROTEIN (MyD116) INTERACTS WITH PP1α IN THE YEAST TWO-HYBRID SYSTEM

The yeast strain Y190 (16) transformed with plasmid pRB4890, and growing on Trp- selective medium was retransformed with a human brain cDNA library (CLONTECH) and subjected to selection on Trp⁻/Leu⁻/His⁻ medium in the presence of 25 mM 3-aminotriazole (Sigma). Moderate to fast growing colonies five days after plating were restreaked on Trp⁻/Leu⁻/His⁻ medium. The library derived plasmids were recovered by transformation of *E*. *coli* HB101 with total yeast DNA preparations, followed by selection on Leu⁻/ampicillin⁺ medium as previously described in Durfee *et al*., *Gene Devel.* **7**:555-569 (1993). The filter assay for β-galactosidase was done as recommended by Clontech.

As discussed briefly above, the two-hybrid system is particularly useful for cloning genes which encode proteins which interact with a protein of interest. See Fields *et al*., *Nature* **340**:245-246 (1989) and Chien *et al*., *Proc*. *Natl*. *Acad. Sci. USA* **88**:9578-9582. The carboxyl terminus of MyD116 was selected as bait for the two-hybrid system because (i) it and γ₁34.5 share amino acid sequence homology [see Chou *et al*., *Proc*. *Natl. Acad. Sci. USA* **91**:5247-5251 (1994) and McGeoch *et al*., *Nature* (London) **353**:609 (1991)], (ii) the carboxyl terminus of MyD116 can substitute functionally for that of the γ₁34.5 protein, and (iii) the codon usage of MyD116, a mammlian gene, is closer to that of yeast than that of HSV-1. Of the 10⁶ yeast colonies screened, only one colony was positive for β-galactosidase expression. The cDNA recovered from this colony was retransformed into yeast strain Y 190 with various control plasmids encoding fusion proteins. These studies indicated that only the carboxyl terminus of MyD116 interacted with the protein encoded by the isolated cDNA. DNA sequence analysis revealed that the isolated cDNA encoded the catalytic subunit of protein phosphatase 1α (PP1α). See Song *et al*., *Gene* **129**:291-295 (1993).

### Example 7

### BOTH MyD116 AND γ₁34.5 PROTEINS BIND PP1α IN VITRO

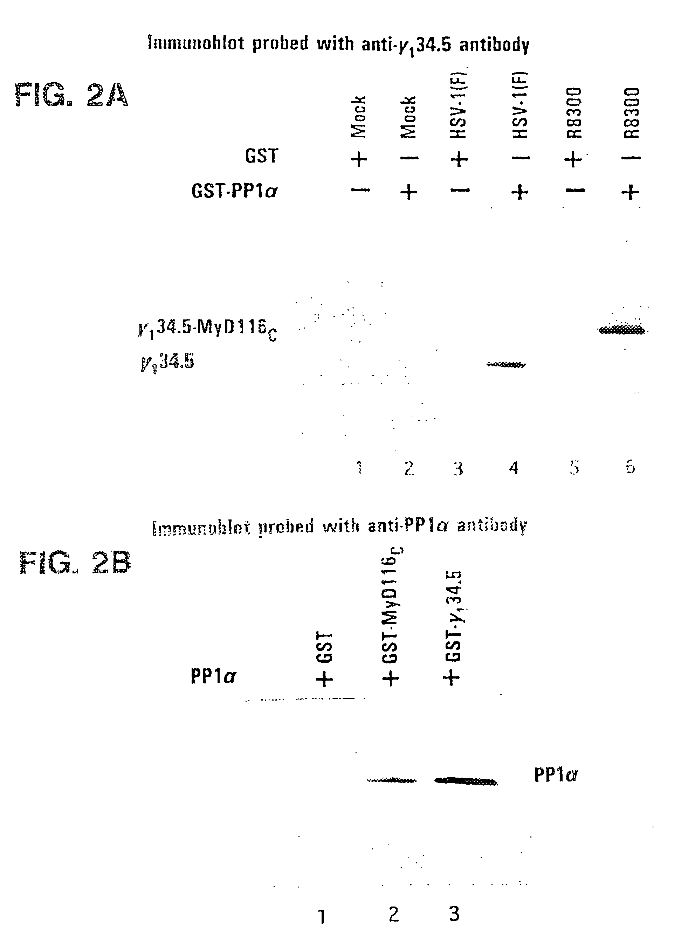

To verify and extend the observations that MyD116 and PP1α interact in yeast, a chimeric GST-PP1α gene constructed as described in Example 3 above was expressed in *E*. *coli.* Replicate HeLa cell cultures were harvested in lysis buffer containing 10 mM Hepes, pH 7.6, 250 mM NaCL, 10mM MgCl₂, 1% Triton X-100, 0.5 mM PMSF and 2mM benzamidine 18 hrs after mock-infection or infection with 10 PFU of HSV-1(F) or R8300 per cell. After 30 min on wet ice and low speed centrifugation to remove nuclei, the supernatant fluids were precleared with GST-beads and then reacted with GST-phosphatase 1 bound beads at 4°C overnight. After extensive rinsing, the proteins bound to beads were solubilized by boiling in disruption buffer containing 50 mM Tris-HCl, pH7.0, 5% β-mercaptoethanol, 2 % SDS, and 2.75 % sucrose, electrophoretically separated on denaturing 12% polyacrylamide gels, transferred to a nitrocellulose sheet, and the blot was probed with anti-γ₁34.5 serum directed to the Ala Thr Pro present in both γ₁34.5 and the chimeric γ₁34.5-MyD116 chimeric protein expressed by R8300. The position of γ₁34.5 protein and of the chimeric protein γ₁34.5-MyD116 are shown in Figure 2A. As shown in Figure 2A, the GST-PP1α protein bound both γ₁34.5 from lysates of HSV-1(F) infected cells and the more slowly migrating γ₁34.5-MyD116 chimeric protein from cells infected with R8300. GST alone did not react with either protein in the appropriate cell lysates.

In a second experiment, purified PP1α was mixed with beads carrying GST or chimeric proteins consisting of GST fused to the amino acids 146 to 263 of γ₁34.5 or to amino acids 485 to 657 of MyD116 as follows.

An aliquot of GST-PP1α fusion protein bound to beads was reacted with 25 units of thrombin (Sigma, St. Louis MO) in PBS saline buffer at room temperature. After eight hrs, the mixture was spun in a table top centrifuge and the supernatant fluid containing PP1α was then dialyzed against lysis buffer, reacted with GST, GST-MyD116(C) or GST-γ₁34.5(C) bound to beads and processed as described in Panel A. PP1 was detected with anti-phosphatase 1α antibody (Upstate Biotechnology Incorporated).

The proteins bound to GST or to the chimeric proteins were solubilized, subjected to electrophoresis on a denaturing gel, and reacted with anti-PP1α antibody. As shown in Figures 2A and 2B, both chimeric proteins but not GST brought down a protein with an apparent Mᵣ of 38,000 (molecular weight markers not shown) and which reacted with the anti-PP1α antibody.

### Example 8

### PHOSPHATASE ACTIVITY IS ESSENTIAL FOR PROTEIN SYNTHESIS IN INFECTED CELLS

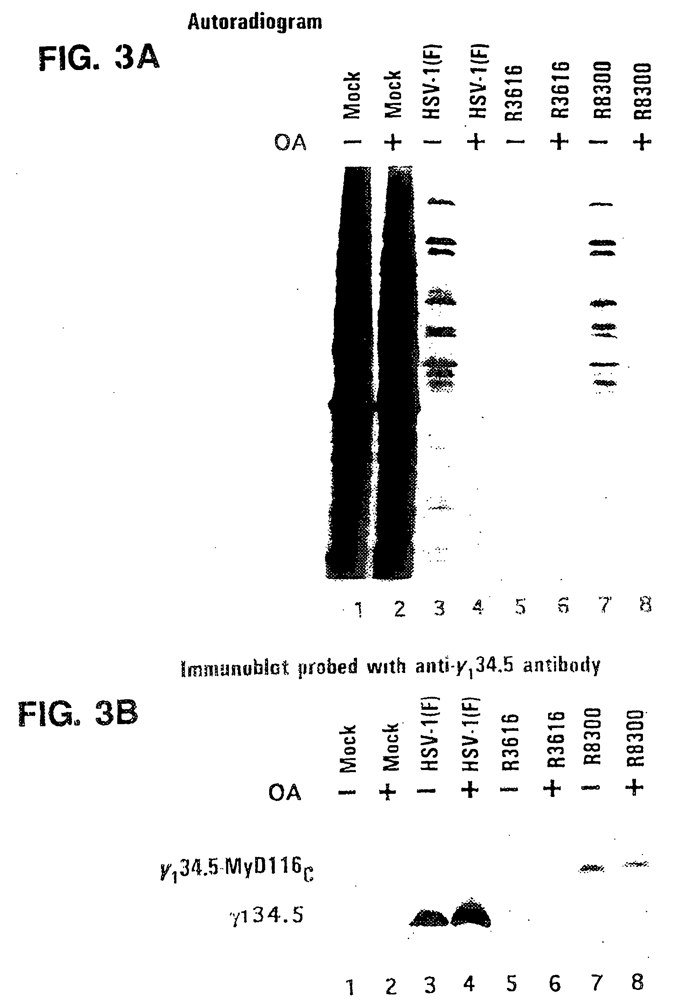

Tests were conducted to determine whether phosphatase activity is essential for sustained protein synthesis in infected cells. [³⁵S]methionine-labeled proteins from cell lysates were infected with the indicated viruses in the presence or absence of okadaic acid (OA). SK-N-SH cells were either mock infected or infected with 20 PFU of HSV-1(F), R3616, or R8300 per cell. At two hrs after exposure to the viruses, the cells were overlaid with medium 199V [Ackermann *et al*., *J. Virol*. **58**:843-850 (1986)] supplemented with or without okadaic acid (25 ng/ml). At 14 hrs after infection, the cells were overlaid for one hr with one ml of medium 199V lacking methionine but supplemented with 50 µCi of [³⁵S]methionine (specific activity, > 1000Ci/mmol; Amersham) for one hr, then harvested, solubilized in disruption buffer, subjected to electrophoresis in denaturing 12 % polyacrylamide gels, transferred to a nitrocellulose sheet and subjected to autoradiography as described elsewhere [Chou *et al*., *Proc. Natl*. *Acad. Sci*. *USA*, **89**:3266-3270 (1992)]. The results (Figure 3A) show okadaic acid totally inhibited protein synthesis in cells infected with wild type HSV-1(F), the γ₁34.5⁻ virus R3616, or the R8300 recombinant expressing the γ₁34.5-MyD116 chimeric protein. In contrast, okadaic acid had no apparent effect on protein synthesis in mock-infected cells.

To test a trivial hypothesis that okadaic acid blocked in some fashion the synthesis of γ₁34.5 or the γ₁34.5-MyD116 proteins in infected cells, the lysates of cells infected with wild type and recombinant viruses were solubilized, subjected to electrophoresis in denaturing gels, electrically transferred to a nitrocellulose sheet and reacted with antibody to the γ₁34.5 protein. As shown in Figure 3B, the lysates of cells infected with HSV-1(F) or with R8300 expressed γ₁34.5 or γ₁34.5-MyD116 protein, respectively. The antibody did not react with lysates of mock-infected cells or cells infected with γ₁34.5⁻ virus.

### Example 9

### THE eIF-2(α³²P) PHOSPHATASE ACTIVITY IS HIGHLY ABUNDANT IN WILD-TYPE OR R8300 RECOMBINANT VIRUS-INFECTED CELLS AND SIGNIFICANTLY DECREASED IN γ₁34.5⁻ VIRUS-INFECTED CELLS

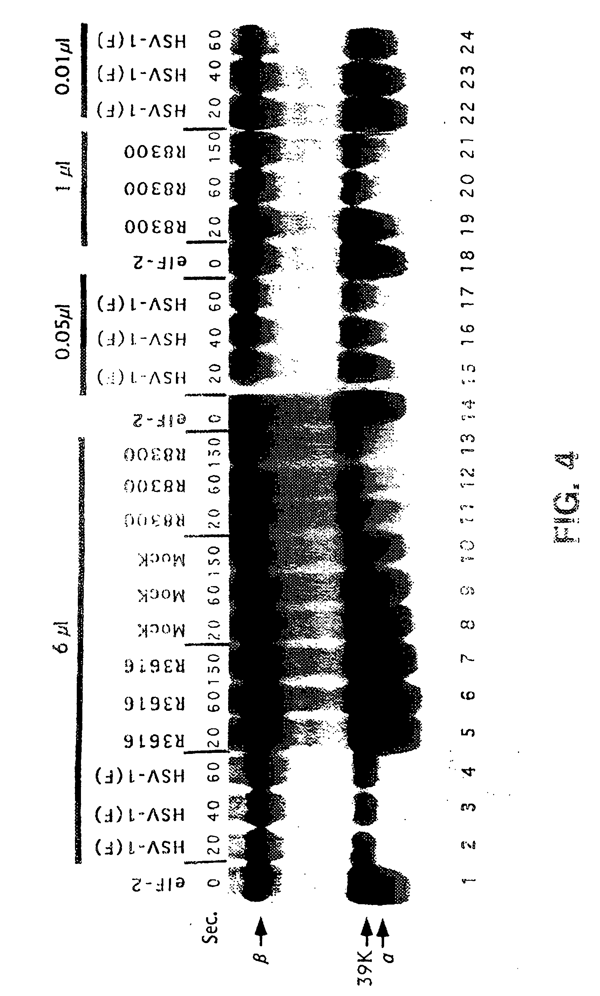

Experiments were conducted to ascertain whether the shutoff of protein synthesis in cells infected with γ₁34.5⁻ virus R3616 was determined by the level of eIF-2α phosphatase activity. S10 fractions were prepared from lysates of HeLa cells either infected or mock-infected and tested for their ability to dephosphorylate eIF-2(α³²P). Unreacted eIF-2(α³²P) (Figure 4, lanes 1, 14, 18) showed three labeled bands representing eIF-2α, eIF-2β (arrows), and a small amount of a M, 39,000 protein (arrow) that is a contaminant distinct from eIF-2α [See, Gross *et al*., *J*. *Biol. Chem*. **255**:6270-6275 (1980)]. Reaction of this eIF-2 with the S10 fraction from mock-infected cells showed a moderate rate of loss of radioactivity from eIF2(α³²P) (Figure 4 and Figures 5A-5D). This eIF-2(α³²P) phosphatase activity was reduced approximately three-fold in cells infected with γ₁34.5⁻ virus. In sharp contrast, the S10 fraction of cells infected with the wild type virus or with the R8300 mutant virus exhibited so marked a level of eIF-2(α³²P) phosphatase activity that virtually all added eIF-2(α³²P) radioactivity was removed within the first 20 seconds of reaction (Figure 4, lanes 2 and 11). To verify that this phosphatase activity does not continue after the reaction mixture is mixed with the SDS denaturing solution, it was tested and found that eIF-2(α³²P) is completely stable following addition to a mixture of SDS and S10 fraction from cells infected with either wild type or R8300 recombinant virus.

To quantify the increase in eIF-2(α³²P) phosphatase activity associated with wild type or R8300 recombinant virus infection of HeLa cells, the S10 fractions from these cells were progressively diluted until the rate of dephosphorylation of eIF-2(α³²P) could be measured accurately. Only when the S10 fraction of wild type infected cells was diluted 600 fold could the eIF-2(α³²P) phosphatase activity be measured accurately, and it was still five fold greater than that of mock-infected cells (Figure 4 and Figures 5A-5D). These findings indicate that the eIF-2(α³²P) phosphatase activity of wild type infected cells increased approximately 3000 fold. Similar analyses indicated that in cells infected with the R8300 recombinant virus this activity increased approximately 50 fold (Figures 4 and 5A-5D). The removal of radioactivity in these samples represented dephosphorylation and not proteolysis inasmuch as it was not associated with loss of eIF-2α protein is measured by staining. In addition, the marked increased in eIF-2(αP) phosphatase activity seen in S10 fractions of wild type virus-infected cells may be specific for eIF-2α inasmuch as neither the M, 39,000 phosphoprotein nor eIF-2(β³²P) was significantly affected under the same conditions (Figure 4). Finally, the results shown in Figures 4 and 5A-5D were reproducible and obtained in two determinations with each of three S10 fraction from mock-infected or infected HeLa cells.

### Example 10

### THE eIF-2(αP) PHOSPHATASE ACTIVITY IS SENSITIVE TO PP1α INHIBITOR 2

To determine whether eIF-2(α³²P) was dephosphorylated by PP1α, the rate of dephosphorylation of eIF-2(α³²P) as a function of the concentration of Inhibitor 2, a known inhibitor of PP1, was measured. As shown in Figure 5, panel B, eIF-2(α³²P) phosphatase in the S10 fraction from wild-type virus- or R8300 virus-infected cells was similarly sensitive to Inhibitor 2 with a 50% reduction in the rate of dephosphorylation attained at approximately 90 and 120 µg of Inhibitor 2/ml, respectively. This suggests that activated eIF-2(α³²P) phosphatase is derived from PP1. However, the concentration of Inhibitor 2 required for inhibition of the activated eIF-2(αP) phosphatase appears to be much higher than that required to inhibit eIF-2(α³²P) phosphatase in mock-infected cells and also much higher than that required to inhibit PP1α in cell extracts. See Cohen, *Methods in Enzymology* **201**:389-398 (1991). This could be due to the possible association of γ₁34.5 with HeLa cell PP1α, resulting in phosphatase activity directed specifically at eIF-2(αP), as suggested by the results shown in Figure 4.

### Example 11

### THE ACTIVATED PHOSPHATASE ACTIVITY IS SPECIFIC FOR eIF-2α

By way of confirmation that eIF-2(α³²P) phosphatase is specifically activated in infected cells, the dephosphorylation of [⁻³²P] phosphorylase, a known substrate of serine/threonine phosphatases, by lysates of HeLa cells mock infected or infected with wild type virus was examined. The results (Figure 5C) showed that the ³²P-phosphorylase phosphatase activity of HSV-1(F)-infected cells. is only one half that of mock-infected cells. The activity responsible for the dephosphorylation of ³²P-phorphorylase in wild type virus infected cells is sensitive to Inhibitor 2 (Figure 5D). The observation that phorphorylase phosphatase activity in wild type virus-infected cells is significantly reduced relative to that of mock-infected cells suggests that the mechanism of activation of eIF-2(αP) phosphatase may involve association of the γ₁34.5 gene product with a portion of the cellular PP1α, converting this fraction to a phosphatase activity that is specific for eIF-2(αP).

### Example 12

### CELL-FREE SYSTEMS FOR IDENTIFICATION OF INDUCERS AND INHIBITORS OF PROGRAMMED CELL DEATH

The observations described above that programmed cell death is associated with phosphorylation of eIF-2α and the shut-off of protein synthesis and that proteins such as γ₁34.5 and GADD (MyD116) prevent the shutoff of protein synthesis by redirecting PP1α to dephosphorylate eIF-2α to enable continued protein synthesis despite the presence of activated PKR, were exploited to prepare cell-free systems for identifying candidate inducers or inhibitors of programmed cell death.

As is evident from the foregoing examples, cell-free systems for identifying inhibitors of programmed cell death according to the invention comprise phosphorylated eIF-2α and PP1α. The eIF-2α may be phosphorylated with ³²P. The system may further comprise a post-ribosomal supernatant of cells such as HeLa cells, SK-N-SH cells or other eucaryotic cells and/or a suitable buffer. A candidate inhibitor of programmed cell death is then introduced into the system and its effect on the level of eIF-2α phosphorylation is determined as described above. Inhibitors of programmed cell death are identified by their ability to decrease the level of phosphorylation of eIF-2α by, for example, redirecting PP1α to dephosphorylate eIF-2α. Similarly, inhibitors of programmed cell death may be identified by their ability to prevent the phosphorylation of eIF-2α. The ability of a candidate inhibitor of programmed cell death may also be identified by its ability to interact with PKR in a cell-free system.

Cell-free systems according to the invention are also useful for identifying inducers of programmed cell death. Thus, such systems comprise eIF-2α, PP1α and a suitable phosphate donor such as ATP or an ATP analog. ATP may be γ³²ATP. The cell-free system may additionally comprise a post-ribosomal supernatant derived from eukaryotic cells. The cell-free system may additionally comprise PKR. Such inducers are identified by their ability to phosphorylate or to maintain the level of phosphorylated eIF-2α by, for example, directing PP1α or PKR to phosphorylate eIF-2α in the presence of a suitable phosphate donor such as ATP or an ATP analog (which may be γ³²P-labeled ATP) when introduced into the cell-free system.

The level of phosphorylation of eIF-2α, and the phosphatase and phosphorylase activities associated with the phosphorylation and dephosphorylation of eIF-2α are measured are described, for example, in Examples 4, 5, and 9 above. The rate of the phosphatase and/or phosphorylase reaction may also be measured and the data analyzed using, for example, Michaelis-Menten analysis in order to more fully characterize and quantitate the respective dephosphorylation and phosphorylation reactions.

In accordance with the invention, varying concentrations of inhibitor or inducer are introduced in the cell-free systems of the invention in order to ascertain the optimal concentrations at which the substances exert their effects. Human PP1α and eIF-2α are preferred in the practice of the present invention. The use of recombinantly produced PP1α and eIF-2α are also within the scope of the invention. Analogs or homologs of eIF-2α and/or PPIα derived from other eukaryotes or prokaryotes are also within the scope of the invention as are eIF-2α and PPIα prepared by recombinant methods.

Phosphorylated (labeled) eIF-2α produced in the cell-free system of the invention may be separated from other components of the system by electrophoresis or by chromatographic methods. By way of example, labeled eIF-2α may be separated on denaturing polyacrylamide gels after which the separated components may be transferred to, for example, a nylon or nitrocellulose membrane followed by exposure to X-ray film. Relative levels of phosphorylation are then determined after developing the exposed X-ray film and quantifying the density of bands corresponding to eIF-2α by, for example, densitometry. The autoradiograph may also be used to localize the bands on the membrane corresponding to labelled eIF-2α after which they may be excised from the membrane and counted by liquid scintillation or other counting methods. eIF-2α and PPIα may be of mammalian origin.

The foregoing was presented by way of non-limiting example and it should not be construed to limit the present invention as set out in the appended claims.

## Claims

1. A method for identifying inhibitors of programmed cell death in a cell-free system, the method comprising the steps of:
(a) preparing a cell-free system comprising phosphorylated eIF-2α and protein phosphatase 1α (PP1α);
(b) introducing into the cell-free system of step (a) a candidate inhibitor of programmed cell death and allowing the resulting mixture to incubate; and,
(c) measuring the level of phosphorylation of eIF-2α resulting from step (b);
wherein an inhibitor of programmed cell death decreases or prevents increase of the level of phosphorylation of eIF-2α that accompanies programmed cell death when compared to a control.

2. The method of claim 1 wherein the cell-free system comprises a post-ribosomal supernatant derived from mammalian cells.

3. The method of claim 2 wherein the post-ribosomal supernatant is derived from mammalian cells infected with a herpes simples virus lacking expressible γ,34.5 genes.

4. The method of claim 1 or 2 wherein the phosphorylated eIF-2α is [³²P]-labeled eIF-2α.

5. The method of claim 1 wherein the phosphorylated eIF-2α is prepared by reaction in a mixture comprising eIF-2α, hemin-controlled translational repressor, and ATP or an analog thereof.

6. A method for identifying inducers of programmed cell death in a cell-free system, the method comprising the steps of:
(a) preparing a cell-free system comprising eIF-2α, protein phosphatase 1α (PP1α), and ATP or an analog thereof;
(b) introducing into the cell-free system of step (a) a candidate inducer of programmed cell death and allowing the resulting mixture to incubate; and,
(c) measuring the level of phosphorylation of eIF-2α resulting from step (b);
wherein an inducer of programmed cell death increases or prevents decrease of the level of phosphorylation of eIF-2α that accompanies programmed cell death when compared to a control.

7. The method of claim 1, 6 or 5 wherein the cell-free system of step (a) as defined in claim 1 or 6 or the mixture of claim 5 further comprises a buffer.

8. The method of claim 6 wherein the ATP is γ³²P-labeled ATP.

9. The method of claim 6 wherein the cell-free system further comprises a post-ribosomal supernatant derived from mammalian cells.

10. The method of claim 6 or 9 wherein the cell-free system further comprises double-strand RNA-dependent protein kinase (PKR).

## Patentansprüche

1. Ein Verfahren zur Erkennung von Inhibitoren des programmierten Zelltodes in einem Zell-freien System, wobei das Verfahren die Schritte umfaßt:
(a) Herstellen eines Zell-freien Systems, das phophoryliertes eIF-2α und Protein-Phosphatase 1α (PP1α) umfaßt;
(b) Einführen eines Inhibitor-Kandidaten des programmierten Zelltodes in das Zell-freie System aus Schritt (a) und Zulassen der Inkubation der resultierenden Mischung; und
(c) Messen des Phosphorylierungsgrades von eIF-2α, das aus Schritt (b) resultiert;
wobei ein Inhibitor des programmierten Zelltodes im Vergleich zu einer Kontrolle den Phosphorylierungsgrad von eIF-2α, der mit dem programmierten Zelltod einhergeht, reduziert oder eine Zunahme verhindert.

2. Das Verfahren aus Anspruch 1, wobei das Zell-freie System einen post-ribosomalen Überstand umfaßt, der aus Säugetierzellen stammt.

3. Das Verfahren aus Anspruch 2, wobei der post-ribosomale Überstand aus Säugetierzellen stammt, die mit einem Herpessimplex-Virus infiziert sind, der keine exprimierbaren γ,34.5 Gene besitzt.

4. Das Verfahren aus Anspruch 1 oder 2, wobei das phosphorylierte eIF-2α mit [³²P]-markiertes eIF-2α ist.

5. Das Verfahren aus Anspruch 1, wobei das phosphorylierte eIF-2α durch Umsetzung in einer Mischung hergestellt wird, die eIF-2α, Hämin-kontrollierten Translations-Repressor, und ATP oder ein Analogon desselben umfaßt.

6. Ein Verfahren zur Erkennung von Induktoren des programmierten Zelltodes in einem Zell-freien System, wobei das Verfahren die Schritte umfaßt:
(a) Herstellen eines Zoll-freien Systems, das eIF-2α, Protein-Phosphatase 1α (PP1α), und ATP oder ein Analogon desselben umfaßt;
(b) Einführen eines Induktor-Kandidaten des programmierten Zelltodes in das Zell-freie System aus Schritt (a) und Zulassen der Inkubation der resultierenden Mischung; und,
(c) Messen des Phosphorylierungsgrades von eIF-2α, das aus Schritt (b) resultiert;
wobei ein Induktor des programmierten Zelltodes im Vergleich zu einer Kontrolle den Phosphorylierungsgrad von eIF-2α, der mit dem programmierten Zelltod einhergeht, erhöht oder eine Abnahme verhindert.

7. Das Verfahren aus Anspruch 1, 6 oder 5, wobei das Zell-freie System aus Schritt (a), wie definiert in Anspruch 1 oder 6, oder die Mischung aus Anspruch 5 weiterhin einen Puffer umfaßt.

8. Das Verfahren aus Anspruch 6, wobei das ATP mit γ³²P-markiertes ATP ist.

9. Das Verfahren aus Anspruch 6, wobei das Zell-freie System weiterhin einen post-ribosomalen Überstand umfaßt, der aus Säugetierzellen stammt.

10. Das Verfahren aus Anspruch 6 oder 9, wobei das Zell-freie System weiterhin eine Doppelstrang RNA-abhängige Proteinkinase (PKR) umfaßt.

## Revendications

1. Procédé pour identifier des inhibiteurs de mort cellulaire programmée dans un système acellulaire, le procédé comportant les étapes consistant à :
(a) préparer un système acellulaire comportant eIF-2α phosphorylée et la protéine phosphatase 1α (PP1α) ;
(b) introduire dans le système acellulaire de l'étape
(a) un inhibiteur candidat de mort cellulaire programmée et faire incuber le mélange résultant ; et,
(c) mesurer le niveau de phosphorylation de eIF-2α résultant de l'étape (b) ;
dans lequel un inhibiteur de mort cellulaire programmée diminue le niveau de phosphorylation de eIF-2α qui accompagne la mort cellulaire programmée, ou inhibe son augmentation, par comparaison à un témoin.

2. Procédé selon la revendication 1, dans lequel le système acellulaire comporte un surnageant post-ribosomique dérivé de cellules de mammifères.

3. Procédé selon la revendication 2, dans lequel le surnageant post-ribosomique est dérivé de cellules de mammifères infectées par le virus de l'herpès simplex ne présentant pas de gènes γ₁34.5 pouvant être exprimés.

4. Procédé selon la revendication 1 ou 2, dans lequel la eIF-2α phosphorylée est eIF-2α marquée par [³²P].

5. Procédé selon la revendication 1, dans lequel la eIF-2α phosphorylée est préparée par réaction dans un mélange comportant eIF-2α, un répresseur de traduction commandé par hemin, et ATP ou un analogue de celui-ci.

6. Procédé pour identifier des stimulateurs de mort cellulaire programmée dans un système acellulaire, le procédé comportant les étapes consistant à :
(a) préparer un système acellulaire comportant eIF-2α, la protéine phosphatase 1α (PP1α), et ATP ou un analogue de celui-ci ;
(b) introduire dans le système acellulaire de l'étape
(a) un stimulateur candidat de mort cellulaire programmée et faire incuber le mélange résultant ; et,
(c) mesurer le niveau de phosphorylation de eIF-2α résultant de l'étape (b) ;
dans lequel un stimulateur de mort cellulaire programmée augmente le niveau de phosphorylation de eIF-2α qui accompagne la mort cellulaire programmée, ou inhibe sa diminution, par comparaison à un témoin.

7. Procédé selon la revendication 1, 6 ou 5, dans lequel le système acellulaire de l'étape (a) comme défini dans la revendication 1 ou 6 ou le mélange selon la revendication 5 comporte de plus un tampon.

8. Procédé selon la revendication 6, dans lequel l'ATP est un ATP marqué par γ³²P.

9. Procédé selon la revendication 6, dans lequel le système acellulaire comporte de plus un surnageant post-ribosomique dérivé de cellules de mammifères.

10. Procédé selon la revendication 6 ou 9, dans lequel le système acellulaire comporte de plus une protéine kinase dépendante d'ARN double brin (PKR).


### SEQUENCE LISTING

(1) GENERAL INFORMATION:
   (i) APPLICANT: ARCH DEVELOPMENT CORPORATION
   (ii) TITLE OF INVENTION: METHODS FOR IDENTIFYING INDUCERS AND INHIBITORS OF PROGRAMMED CELL DEATH
   (iii) NUMBER OF SEQUENCES: 4
   (iv) CORRESPONDENCE ADDRESS:
      (A) ADDRESSEE: Marshall, O'Toole, Gerstein, Murray & Borun
      (B) STREET: 233 South Wacker Drive/6300 Sears Tower
      (C) CITY: Chicago
      (D) STATE: Illinois
      (E) COUNTRY: United States of America
      (F) ZIP: 60606-6402
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/795,470
      (B) FILING DATE: 04-FEB-1997
   (viii) ATTORNEY/AGENT INFORMATION:
      (A) NAME: Zeller, James P.
      (B) REGISTRATION NUMBER: 28,491
      (C) REFERENCE/DOCKET NUMBER: 27373/33742 PCT
   (ix) TELECOMMUNICATION INFORMATION:
      (A) TELEPHONE: (312) 474-6300
      (B) TELEFAX: (312) 474-0448
      (C) TELEX:
(2) INFORMATION FOR SEQ ID NO:1:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:1:
   TGACTGGATG CAGAGGCGGC TCAGATTGTT C 31
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:2:
   AGCGCGCAAT TAACCCTCAC TAAAG 25
(2) INFORMATION FOR SEQ ID NO:3:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:3:
   GCACTGAATT CTCCGACAGC GAGAAGCTCA AC 32
(2) INFORMATION FOR SEQ ID NO:4:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:4:
   GCACTGTCGA CATCTGGGGC ACAGGGTGGT GT 32